# EUROPEAN PATENT APPLICATION

(11) **EP 2 295 394 A1**
(43) Date of publication of application: **16.03.2011**
(21) Application number: 09170121.9
(22) Date of filing: 11.09.2009
(51) Int. Cl.: C07C 29/92, C07C 67/03, C07C 67/08, C07C 67/54, C07C 69/14, C07C 69/24, C07C 69/18, C07C 69/30, C07C 31/22

(54) **Process for the purification of crude glycerol**

(71) Applicant: Rhodia Poliamida E Especialidades Ltda, Sao Paulo - SP (BR)
(72) Inventor: Lourenco, Wagner Celio Ferraz, 130783-130, Campinas (SP) (BR); Macret, Richard, 01232-010, Soa Paulo (SP) (BR); Cielo, Jose Eduardo, 13106-110, Sousas (SP) (BR)
(74) Representative: Boittiaux, Vincent

(57) **Abstract**

The present invention generally concerns a process for the purification of crude glycerol obtained as a by-product of industrial processes such as saponification, hydrolysis and transesterification with low chain alcohols of natural oils and fats. The present invention particularly concerns the purification treatment of crude glycerol obtained as a by-product of the biodiesel production from vegetable oils and animal fats, aiming to obtain a concentrated purified product.

## Description

The present invention generally concerns a process for the purification of crude glycerol obtained as a by-product of industrial processes such as saponification, hydrolysis and transesterification with low chain alcohols of natural oils and fats. The present invention particularly concerns the purification treatment of crude glycerol obtained as a by-product of the biodiesel production from vegetable oils and animal fats, aiming to obtain a concentrated purified product.

### BACKGROUND OF THE INVENTION

In the text that follows, the mention to glycerol as a by-product of the biodiesel production is merely ilustrative of glycerol obtained from any transformation process of vegetable oils or animal fat, where glycerol is a by-product.

A known process for the production of biodiesel involves the transesterification of triglyceride-containing vegetable oils (such as coconut, soy, castor, sunflower and peanut) or animal fat, with a short chain alcohol, in general methanol or ethanol, for instance in the presence of a homogeneous or heterogeneous alkaline catalyst.

In such processes, fatty acid alkyl esters are obtained from the triglyceride comprised in the natural oils and fats utilized as raw material, with the concomitant generation of crude glycerol as by-product. A general equation for that reaction would be:
triglyceride + 3 alkyl alcohol → glycerol + 3 fatty acid alkyl ester

Such triglycerides are transesterified with low chain alcohols in the presence of an alkaline catalyst such as hydroxides or alkoxides, in a homogeneous or heterogeneous catalysis.

Typically, an excess of alkyl alcohol is used in the transesterification reaction. After reaction completion, crude glycerol is separated from the biodiesel phase, for example by decantation. Usually 1000 kg of raw material results in 90% biodiesel and 10% crude glycerol.

The immiscible crude glycerol phase frequently has a variable glycerol content as the major component, as well as the excess alkyl alcohol used for the transesterification reaction, besides glycerides and glyceride-like compounds, some of the alkyl esters, inorganic salts and water.

After recovery of the excess alkyl alcohol by evaporation, the remaining crude glycerol typically contains 30-88% glycerol, 3-20% water, non-glycerol organic material (NGOM), 1-10% inorganic salts (commonly sodium or potassium salts), residual fatty acids, glycerides and a small amount of alcohol.

Typical samples of crude glycerol obtained from different production processes and starting raw materials are shown on Table I below, with large observed differences in levels of impurities and quality.

**Table I: Typical glycerol percentage and main impurities of crude glycerol from biodiesel processes.**

| ANALYSIS | Sample 1 | Sample 2 | Sample 3 | Sample 4 | Sample 5 |
|---|---|---|---|---|---|
| Glycerol (% w/w) | 81,1 | 81,0 | 82,7 | 80,0 | 82,4 |
| Water (% w/w) | 8,3 | 7,4 | 9,3 | 13,2 | 6,9 |
| Methanol (% w/w) | 1,4 | 2,0 | 1,7 | 1,1 | 1,1 |
| (NGOM) (%w/w) | 6,5 | 8,1 | 1,7 | 0,7 | 2,5 |
| Ester (% w/w) | 9,2 | 4,9 | - | - | - |
| Ash (% w/w) | 4,2 | 3,5 | 6,3 | 6,1 | 8,2 |
| Combined Alkalinity (mg KOH/g) | * | * | 0,7 | 0,8 | 0,6 |
| Acidity (mg KOH/g) | 8,7 | 4,7 | 0,2 | 1,3 | 0,1 |
| Volatile 100 oC (% w/w) | 9,7 | 9,4 | 11,0 | 14,3 | 8,0 |
| pH (10% Aqueous Solution) | 2,3 | 6,5 | 7,1 | 6,2 | 6,6 |

| | | | | | |
|---|---|---|---|---|---|
| (*) = below the detection limit | | | | | |

This variation in the composition of crude glycerol obtained from biodiesel manufacturing processes, associated with the presence of impurities, especially inorganic salts, creates difficulties for the use of such a glycerol as raw material for industrial processes or its use as such.

A number of techniques for the purification of crude glycerol has been described in the technical literature comprising steps such as conventional filtration, ultra filtration using membranes, distillation, treatment with ion exchange resins, various chemical treatments, and so forth.

A common process for the purification of crude glycerol is by distillation, requiring specially careful controlled conditions since glycerol submitted to high temperatures, in the order of 170-180 °C, can decompose or polymerize generating the formation of undesirable impurities and a consequent loss in the yield of recovered glycerol.

Furthermore, the purification of crude glycerol by distillation requires expensive processing equipments, costly and energy intensive.

There are many known purification processes for crude glycerol described in the technical literature, such as the ones exemplified in Ullman's Encyclopaedia of Chemical Technology, Volume A12, Page 481.

In the patent document US4655879 it is described a very laborious purification process for crude glycerol, involving a large number of steps, where crude glycerol is alkalinized in the presence of air for oxidation, distilled at 165-180 °C and 10-20 mbar in a thin-layer evaporator. After that, the residue is further distilled, followed by a rectification in a low-pressure-drop column in a falling-film evaporator and treatment with activated carbon for colour removal.

In the Brazilian patent document P18904007 the crude glycerol is purified with a combination of operations such as adjustment of the pH in the range of 9 to 12, heating at 60-100 °C, followed by micro filtration and ultra filtration. The obtained glycerol containing water is distilled to produce a technical grade glycerol or submitted to an ion-exchange treatment before distillation to obtain a glycerol of pharmaceutical grade.

In another example, in the patent document US 7126032, the crude glycerol having pH of approximately 11,6 is acidified to pH between 5,8 and 6,8, is heated to 125 - 160 °C in the presence of a nitrogen sparging flow for the elimination of alcohol, water and non-glycerol organic products. Further it is distilled in a flash column with temperatures in the upper zone of 156 to 160 °C and the lower zone maintained at 173 to176 °C. The absolute pressure in the system is maintained at 7 to 9 mm Hg. The side product was a lightly amber colour product and the overhead product a water white product with a total recovery of 50%.

The patent document EP0358255 describes a process for purifying crude glycerol obtained from high pressure splitting of oils and fats using a microfiltration step on alumina and/or zirconia supported in a ceramic material. Additional steps such as distillation and/or treatment with exchange resin are needed and if crude glycerol from for example biodiesel process is to be purified, a water dilution is necessary for the described process.

The process described in patent document WO 2008/156612 comprises the filtration of crude glycerol compositions through two or more nanofiltration systems or reverse osmosis filters connected in series. At least one of the filters comprises filtering media having a contact angle between about 44 and about 56 degrees.

The Brazilian patent document BR 0704952 describes a process for crude glycerol purification based on gel ion exchange resins, strong acidic ion exchange resins with a further step of regeneration as cited on reference J. Chem. Technol. Biotechnol., 2009, 84, 738-744 and chemical treatment as described in the patent document US 7534923. The chemical treatment uses 12% of sodium borohydride in 40% caustic solution and pH 8-9. Besides using a chemical treatment, the obtained mixture has to be further treated by ion exchange chromatography to eliminate the sodium salts formed.

In view of that prior art, it is desirable to develop a simple purification process for the crude glycerol coming from industrial processes, particularly from the biodiesel production, with a reduced number of operations, of relatively low cost and using milder conditions of temperature and pressure, to generate a purified glycerol having adequate quality for a range of application.

### DESCRIPTION OF THE INVENTION

The present invention concerns a process for the purification of crude glycerol characterized by the fact that it comprises the following steps:
(a) reacting crude glycerol with lower carboxylic acids or anhydrides;
(b) separating the formed glycerol esters, for instance by distillation;
(c) reacting the formed esters with at least one alkyl or cycloalkyl alcohol;
(d) separating the formed glycerol.

By crude glycerol it is meant the glycerol that is a by-product of industrial processes such as saponification, high pressure hydrolysis or transesterification with alcohols of natural oils and fats, particularly crude glycerol recovered as a by-product from the biodiesel production from vegetable oils and/or animal fat.

The process of this invention is suitable for the purification of crude glycerol concentrations in the range of 5 to 95% in weight, particularly in the typical range as obtained in the biodiesel production, such as 40 to 90% glycerol, independently of water, salts and other usual by-products from the biodiesel production process.

Step (a) is performed with or without a catalyst to obtain a mixture of glycerol esters. The original possibly alkaline pH of crude glycerol, the raw material for this step, is acceptable, although a pre-adjustment of the pH to acidic (about 2.5) to neutral (about 7.0) is preferred, particularly in the range of 5,0 to 7,0.

According to the characteristics of the crude glycerol, the slightly acidified glycerol solution may be filtered to remove insoluble organic matter, prior to step (a).

The esterification reaction of step (a) can be best carried out using C1 to C6 alkyl carboxylic acids and/or their anhydrides, particularly C1 to C4 carboxylic acids and/or anhydrides such as formic, acetic, propionic or butyric and, still more particularly, acetic acid and/or anhydride.

Acetic anhydride is advantageously used as esterification agent in step (a) to favor the production of purer glycerol triacetate (1,2,3-propanetriol triacetate), usually referred as triacetin, as compared to the use of acetic acid as esterification agent, which favors the production of a mixture of acetins (mono-, di- and triacetates of glycerol). The proportion among the components of this acetate mixture can be effected depending on whether a catalyst is or is not used in the esterification reaction. In this text, the mention to a mixture of glycerol esters, for instance acetates, also encompasses a mixture with almost only one glycerol ester, for instance, triacetin.

Adequate catalysts for the esterification reaction in step (a), not excluding any other, are chosen from inorganic acids such as sulphuric and hydrochloric acids, and sulphonic acids such as methanesulphonic, xylenesulphonic and p-toluenesulphonic acids for the homogeneous catalysis. For heterogeneous catalysis, acidic ion exchange resin may be adequately used as catalyst as well as other solid acidic materials.

The glycerol ester mixtures (for instance glycerol acetate mixtures) and/or the purer glycerol triester (particularly glycerol triacetate), obtained in the esterification reaction of step (a) are separated in step (b), particularly by distillation, typically providing a product containing 98 to 99% of glycerol esters, for instance glycerol acetates.

In step (c), the glycerol ester mixture or glycerol triester can be easily transesterified using C1 to C6 alkyl alcohols, particularly C1-C3 alkyl alcohols such as methanol, ethanol, iso or n-propanol , or C5 or C6 cyclic alcohols such as cyclopentanol or cyclohexanol, in the presence of alkaline or acidic catalysts.

Examples of catalysts for the transesterification reaction of step (c), without excluding any other, are chosen from bases such as sodium or potassium hydroxides, alkoxides such as sodium or potassium methoxide, alkaline ion exchange resins, inorganic acids such as sulphuric and sulphonic acids and acidic ion exchange resins.

An adequate catalyst percentage is 6-10% on weight, based on the glycerol ester mixture mass, particularly 4- 6% and more particularly 1-3 %.

The transesterification reaction of step (c) generates a mixture of glycerol and the corresponding ester of the utilized alcohol, as well as some of the unreacted alcohol.

In step (c), an adequate molar ratio between the amount of the short alkyl alcohol to the amount glycerol ester mixture is 10:1, particularly 7:1 and even more particularly 5:2.

In step (c), the alcohol and the catalyst can be added to the reaction media in one portion or divided in 2 to 5 portions, particularly in 2 or 3 portions.

In step (d), the ester formed in the transesterification reaction of step (c), for example ethyl acetate, and the unreacted excess alcohol, for example ethanol, are eliminated from the reaction mixture by simple distillation at low temperatures, optionally using a nitrogen sparging flow to aid in the removal of both the ester and the alcohol, and glycerol with high purity is obtained, adequate for a large range of applications .

The process of the invention, for purifying crude glycerol compositions, may be performed in batch operations as well as in a continuous process, using either homogeneous or heterogeneous catalysis.

The process of this invention presents advantages over a simple distillation of crude glycerol as the boiling point of glycerol acetates are lower than the one of glycerol, providing milder operational conditions, favoring minimal to no degradation of glycerol with very high overall glycerol conversion, performed with traditional equipment without investments in special equipment, what translates to economic advantages with respect to prior art processes.

The process of this invention also presents advantages over the direct distillation processes of the prior art, since many or most impurities of crude glycerol are left behind when the intermediate glycerol acetates are distilled and isolated. Those impurities, in many processes described in the prior art, are carried along with the glycerol during distillation and have to be removed by a further distillation step that contributes to a more costly and laborious process.

Another advantage of the process of this invention is that in step (a) the ester or acetyl group formed in the esterification of glycerol blocks one to three hydroxyl groups of glycerol which avoids the undesirable side reactions and also the polymerization of glycerol during distillation.

In a preferential manner the process of this invention uses acetic acid or acetic anhydride for the esterification reaction of crude glycerol compositions, in step (a).

According to a particular embodiment of the present invention, as a function of the desired reaction time and productivity of glycerol esters, the water formed during the esterification reaction in step (a) can be azeotropically removed, for instance by simple distillation at atmospheric pressure, for example by using esters of lower alcohols such as ethyl, iso-propyl, n-propyl, n-butyl, iso-butyl, sec-butyl acetate and particularly ethyl, iso-propyl and n-butyl acetates.

According to the invention, the claimed process typically provides glycerol purified to a 95-99,5% grade. The process of this invention has the advantage that, for the transesterification reaction with lower alcohols, the glycerol ester mixtures (particularly mixtures of mono-, di- and tri-acetates, with different contents of each acetate) may present any proportion of such esters.

### EXAMPLES

The following examples are given only as particular embodiments of the invention, and do not in any way limit the extension of the invention to just just what they disclose.

Table II below show typical examples of glycerol ester mixtures obtained with the transesterification in step (a) of the process of the invention, as set forth above, when the lower carboxylic acid is acetic acid and/or acetic anhydride.

**Table II: Characteristics of glycerol acetate mixtures**

| Sample ► | Sample 1 | Sample 2 | Sample 3 | Sample 4 |
|---|---|---|---|---|
| Component▼ | % w/w | | | |
| Triacetin | 48,7 | 82,8 | 61,9 | 99,1 |
| Diacetin | 35,2 | 15,7 | 26,3 | - |
| Monoacetin | 15,3 | 0,6 | 10,5 | - |
| Water | 0,05 | 0,03 | 0,11 | 0,15 |
| Acetic Acid | 0,05 | 0,04 | 0,09 | 0,10 |
| Others | 0,70 | 0,83 | 1,1 | 0,65 |

In the examples that follow, steps (a) and (b) of the process of the invention were already performed.

### Example 1

The glycerol ester mixture of sample 1 of table II (300 g), anhydrous ethanol (270 g) and a solution of 30% sodium methoxide in methanol (2,4 g) were heated at reflux for 4 hours. After 4 hours reflux, the ethyl acetate/ethanol mixture was distilled at 69,8 °C (top temperature) and 78 to 85 °C (reaction temperature). At the end of distillation, the reaction mixture was kept at 85 °C under vacuum of 80-100 mmHg to eliminate residual ethanol and ethyl acetate. 148,3 g of a viscous clear liquid were obtained, containing 87,5% of glycerol, 7,5% glycerol acetate and 0,6% glycerol diacetate as analyzed by gas chromatography and titration.

### Example 2

The glycerol ester mixture of sample 2 of table II (500 g), methanol (230 g) and a solution of 30% sodium methoxide in methanol (4,0 g) were heated at reflux for 40 minutes. The methyl acetate/methanol mixture was distilled at 51,9 to 54,5 oC (top temperature) and 56 to 80 oC (reaction temperature). At the end of distillation, the reaction mixture was kept at 80 oC under vacuum of 80-100 mmHg to eliminate residual methanol and methyl acetate. 156,4 g of a viscous clear liquid were obtained containing 82,1% of glycerol and 16,9% glycerol acetate mixture as analyzed by gas chromatography and titration.

### Example 3

The glycerol ester mixture of sample 1 of table II (405 g), anhydrous ethanol (264 g) and a solution of 30% sodium methoxide in methanol (2 g) were heated at reflux for 40 minutes. The ethyl acetate/ethanol mixture was than distilled at 69,5 oC (top temperature) and 88 to 90 oC (reaction temperature). At the end of distillation, anhydrous ethanol was added (142 g) and catalyst (0,8 g) and the reaction mixture heated to reflux for additional 40 to 45 minutes. The ethyl acetate/ethanol mixture was distilled at 71 to 73 oC (top temperature) and 80 to 90 oC (reaction temperature). At the end of distillation, the reaction mixture was kept at 85-94 oC under vacuum of 80-100 mmHg for a short period of time. 216,5 g of a viscous clear liquid were obtained containing 92,5% of glycerol and 6,5% of ethanol as analyzed by gas chromatography and titration.

### Example 4

The glycerol ester mixture of sample 1 of table II (1000 g), methanol (460 g) and a solution of 30% sodium methoxide in methanol (5 g) were heated at reflux for 20 minutes. The methyl acetate/methanol mixture was then distilled at 51,9 to 54,5 oC (top temperature) and 65 to 80 oC (reaction temperature). At the end of distillation, methanol (330 g) and catalyst sodium methoxide (2 g) were added and the reaction mixture heated to reflux for additional 30 to 35 minutes. The methyl acetate/methanol mixture was distilled at 60,4 °C (top temperature) and 70 to 75 °C (reaction temperature). At the end of distillation, the reaction mixture was kept at 75-80 °C under vacuum of 80-100 mmHg for a short period of time. 524,5 g of a viscous yellowish clear liquid were obtained, containing 94,3% of glycerol and 4,9% of methanol as analyzed by gas chromatography and titration.

### Example 5

The glycerol ester mixture of sample 1 of table II (2000 g), methanol (800 g) and a solution of 30% sodium methoxide in methanol (37 g) were heated at reflux for 20 minutes. At the end of this time, the methyl acetate/methanol mixture was distilled and the procedure repeated two more times adding methanol/sodium methoxide (600/8 g) and (500/8 g). After complete distillation of all methanol/methyl acetate formed, vacuum of 20-40 mmHg was applied to the reaction mixture under heating (85-90 °C) until complete elimination of residual methanol and methyl acetate. A yellowish clear viscous liquid was obtained (998 g) which after titration analysis showed 99,1% of glycerol.

### Example 6

In this experiment, anhydrous ethanol was used instead of methanol following the same procedure as example 5. The starting mass of glycerol ester mixture was 550 g (sample 2) and the anhydrous ethanol/catalyst being added in three portions followed by distillation after each addition: (1) anhydrous ethanol 275g / 10g of sodium methoxide, (2) anhydrous ethanol 220g / 2,2g sodium methoxide and 3) anhydrous ethanol 140g / 2,2g sodium methoxide. After complete elimination of residual ethanol and ethyl acetate at a vacuum of 20-40 mmHg, a yellowish clear viscous liquid was obtained (243,5 g) which after titration analysis showed 98,5% of glycerol.

### Example 7

In another experiment, in a reactor equipped with a pump for continuous addition of alcohol, one added the glycerol ester mixture as described on Table II - sample 2 (1000 g), ethanol (300 g) and a solution of 30% sodium methoxide in methanol (18 g). The reaction mixture was heated at reflux for 20 minutes. At the end of this time, the distillation of the ethyl acetate/ethanol mixture was started while starting the feeding of a solution of anhydrous ethanol (900 g) and sodium methoxide (18 g) at approximately the same rate of the liquid being distilled. After the complete feeding of the ethanol/catalyst solution, the distillation continued under heating (85-90 °C) and vacuum of 20-40 mmHg to complete elimination of residual ethanol and ethyl acetate. A yellowish clear viscous liquid was obtained (440 g) which after titration analysis showed 99,0 % of glycerol.

It is well understood that a person skilled in the art, with the help of the teachings brought herein, is able to perform embodiments of the invention not expressly described in this text, with substantially the same function to reach substantially the same results, but such procedure is equivalent to the invention, therefore being encompassed by the claims presented further on.

## Claims

1. A process for the purification of crude glycerol **characterized by** the fact that it comprises the following steps:
a. reacting crude glycerol with lower carboxylic acids or anhydrides;
b. separating the formed glycerol esters;
c. reacting the formed esters with at least one alkyl or cycloalkyl alcohol;
d. separating the formed glycerol.

2. A process for the purification of crude glycerol according to claim 1 **characterized by** the fact that said crude glycerol is a by-product of industrial processes such as saponification, high pressure hydrolysis or transesterification with alcohols of natural oils and fats.

3. A process for the purification of crude glycerol according to claim 1 **characterized by** the fact that said crude glycerol is a by-product from the biodiesel production from vegetable oils and/or animal fat.

4. A process for the purification of crude glycerol according any of the preceding claims **characterized by** the fact that said crude glycerol is present in a concentration from 5 to 95%, particularly from 40 to 90%, in weight.

5. A process for the purification of crude glycerol according to any of the preceding claims **characterized by** the fact said crude glycerol is pre-adjusted to pH from 2.5 to 7.0, particularly from 5.0 to 7.0.

6. A process for the purification of crude glycerol according to any of the preceding claims **characterized by** the fact that said crude glycerol is filtered, prior to step (a) of claim 1.

7. A process for the purification of crude glycerol according to any of the preceding claims**characterized by** the fact that the separation of step (b) is performed by distillation.

8. A process for the purification of crude glycerol according to any of the preceding claims**characterized by** the fact that the alcohol utilized in step (c) is a C1 to C6 alkyl alcohols, or a C5 or C6 cyclic alcohol.

9. A process for the purification of crude glycerol according to any of the preceding claims **characterized by** the fact that the alcohol utilized in step (c) is a C1-C3 alkyl alcohol, particularly methanol, ethanol, iso or n-propanol.

10. A process for the purification of crude glycerol according to any of claims 1 to 8 **characterized by** the fact that the alcohol utilized in step (c) is a C5 or C6 cyclic alcohol, particularly cyclopentanol or cyclohexanol.

11. A process for the purification of crude glycerol according to any of the preceding claims **characterized by** the fact that the reaction in step (c) is performed in the presence of alkaline or acidic catalysts.

12. A process for the purification of crude glycerol according to claim 11 **characterized by** the fact that the catalysts are chosen from bases, particularly sodium or potassium hydroxides, alkoxides particularly sodium or potassium methoxide, alkaline ion exchange resins, inorganic acids, particularly sulphuric and sulphonic acids and acidic, and ion exchange resins.

13. A process for the purification of crude glycerol according to one one of claims 11 or 12 **characterized by** the fact that the catalyst percentage is 6-10% on weight, based on the glycerol ester mixture mass, particularly 4- 6%, and more particularly 1-3 %.

14. A process for the purification of crude glycerol according to any of the preceding claims **characterized by** the fact that the molar ratio between the amount of the alkyl or cycloalkyl alcohol to the amount glycerol ester mixture in step (c) is 10:1, particularly 7:1 and more particularly 5:2.

15. A process for the purification of crude glycerol according to any of the preceding claims**characterized by** the fact that the separation in step (d) is distillation.

16. A process for the purification of crude glycerol according to any of the preceding claims**characterized by** the fact that the water formed during the esterification reaction in step (a) is azeotropically removed by distillation at atmospheric pressure, particularly using esters of lower alcohols chosen from ethyl, iso-propyl, n-propyl, n-butyl, iso-butyl, sec-butyl acetate, more particularly chosen from ethyl, iso-propyl and n-butyl acetates.
